# FASCICULE DE BREVET EUROPEEN

(11) **EP 0 736 301 B1**
(45) Date de publication et mention de la délivrance du brevet: **22.03.2000**
(21) Numéro de dépôt: 95400774.6
(22) Date de dépôt: 07.04.1995
(51) Int. Cl.: A61K 31/60

(54) **Association d'acétylsalicylate de DL-lysine, de vitamine E et de bêta-carotène**
Kombinationspräparat von Acetylsalicylat von Lysin, Vitamine E und Beta-Karotin
Pharmaceutical composition containing lysine acetylsalicylate, vitamin E and beta-carotene

(43) Date de publication de la demande: 09.10.1996
(73) Titulaire: Sanofi-Synthélabo, 75013 Paris (FR)
(72) Inventeur: Scheldewaert, Rudy, F-78450 Chavenay (FR)
(74) Mandataire: Thouret-Lemaitre, Elisabeth

(56) Documents cités:
- FR-A- 2 115 060
- FR-A- 2 714 292
- THROMB HAEMOSTASIS,, VOL. 49, NO. 2, PAGE(S) 73-77, 1983 STEINER M 'EFFECT OF ALPHA TOCOPHEROL ADMINISTRATION ON PLATELET FUNCTION IN MAN'
- ATHEROSCLEROSIS,, VOL. 82, NO. 3, PAGE(S) 247-252, 1990 VIOLI F et al 'INHIBITION OF CYCLOOXYGENASE-INDEPENDENT PLATELET AGGREGATION BY LOW VITAMIN E CONCENTRATION'
- CLIN. CARDIOL. (USA), VOL. 16, NO. 4 SUPPL. I PAGE(S) I16-I18, Avril 1993 Steiner M. 'Vitamin E: More than an antioxidant'

## Description

La présente invention a pour objet une association d'acétylsalicylate de DL-lysine, de vitamine E et de β-carotène.

La source de vitamine E utilisée est l'α-tocophérol racémique ou dextrogyre, naturel ou synthétique, sous forme de base ou d'un sel tel que l'acétate, le palmitate ou le succinate.

L'acide acétylsalicylique est un agent antiagrégant plaquettaire connu, dont l'utilisation dans la prévention secondaire des évènements cardio- et cérébro-vasculaires a été décrite par exemple dans B.M.J., 436-440 (1974), J.A.M.A., vol. 260, n° 14, 2088-2093 (1988) et Circulation, vol. 87, n° 2, 659-675 (1993).

L'acétylsalicylate de lysine, forme soluble dans l'eau et stable, améliore considérablement la tolérance digestive locale de l'acide acétylsalicylique.

La vitamine E a également un effet antiagrégant plaquettaire. De plus, elle a un effet vasodilatateur de la paroi artérielle.

La vitamine E et le β-carotène sont connus par ailleurs comme agents antioxydants et piégeurs de radicaux libres.

Or, on sait que les radicaux libres provoquent des altérations des acides nucléiques, des protéines et des lipides cellulaires et jouent donc un rôle dans la cancérogenèse, l'athérogenèse et l'ischémie.
On sait que la formation d'athéromes est favorisée par le LDL cholestérol oxydé, du fait de son action inhibitrice de la motilité des macrophages tissulaires, de son incorporation aux monocytes dans la paroi artérielle, de son action cytotoxique vis à vis des cellules endothéliales et de son effet vasoconstricteur artériel et par les radicaux libres libérés lors de traumatismes de l'endothélium.

La vitamine E et le β-carotène, en s'opposant à l'oxydation du LDL cholestérol et en inactivant les radicaux libres libérés par l'endothélium, préviennent ou réduisent la formation d'athéromes et diminuent ainsi les risques de maladies cardio- et cérébro-vasculaires.

Atherosclerosis, vol.82 No.3, pages 247-252 (1990) décrit une étude clinique suggérant l'utilisation d'une combinaison d'aspirine et de vitamine E pour inhiber l'agrégation plaquettaire.

D'autre part, la vitamine E et le β-carotène, en tant qu'antioxydants et piégeurs de radicaux libres, régulateurs de la différenciation cellulaire et stimulants de l'immunité, et la vitamine E, par son effet bloquant de la transformation des nitrosamines en nitrosamides cancérigènes, ont une activité antinéoplasique.

L'association de l'invention est donc destinée à la prévention primaire des maladies cardio- et cérébro-vasculaires, à la prévention secondaire d'événements cardio- et cérébro-vasculaires, à la prévention de la resténose après angioplastie coronarienne, à la prévention de l'apparition et/ou de la progression de lésions athéromateuses, à la prévention de l'athéromatose coronarienne et de la sténose des greffons après pontage coronarien et à la prévention des cancers de tous types.

L'association de l'invention peut être utilisée, en combinaison avec des excipients appropriés, pour la préparation de compositions pharmaceutiques destinées à l'administration par voie orale.

Les excipients sont choisis parmi les composants utilisés habituellement lors de la formulation et de la fabrication des médicaments, tels que diluants, substances tampons, agents de glissement, aromatisants, édulcorants.

Les compositions pharmaceutiques de l'invention peuvent être présentées sous forme de comprimés, de gélules, de capsules ou de poudre, en particulier de poudre pour solution buvable.

Elles peuvent contenir de 54 à 540 mg d'acétylsalicylate de DL-lysine, soit l'équivalent de 30 à 300 mg d'acide acétylsalicylique, de 10 à 1000 UI de vitamine E et de 5 à 50 mg de β-carotène par dose unitaire.

Les compositions de l'invention sous forme de poudre sont obtenues par mélanges successifs des différents constituants à sec dans un mélangeur à poudre.

La poudre peut ensuite être transformée en comprimés ou introduite dans des gélules, des capsules ou des sachets dose unitaire selon des méthodes connues en soi.

L'exemple suivant illustre l'invention :

Exemple 1 : poudre pour solution buvable

| | |
|---|---|
| acétylsalicylate de DL-lysine | 320 mg |
| acétate de DL-α-tocophérol | 250 UI |
| β-carotène | 25 mg |
| Glycine | 35,5 mg |
| Arome | 30 mg |
| | |
| pour un sachet | |

La posologie journalière est de 54 à 540 mg d'acétylsalicylate de DL-lysine, soit l'équivalent de 30 à 300 mg d'acide acétylsalicylique, de 10 à 1000 UI de vitamine E et de 5 à 50 mg de β-carotène.

## Revendications

1. Composition pharmaceutique caractérisée en ce qu'elle est constituée d'un mélange d'acétylsalicylate de D,L-lysine, d'α-tocophérol racémique ou dextrogyre, sous forme de base ou d'un sel, et de β-carotène, en association avec des excipients appropriés.

2. Composition pharmaceutique selon la revendication 1, caractérisée en ce que l'α-tocophérol est utilisé sous forme d'acétate, de palmitate ou de succinate.

3. Composition pharmaceutique selon l'une des revendications 1 ou 2, caractérisée en ce qu'elle contient 54 à 540 mg d'acétylsalicylate de DL-lysine, 10 à 10000 UI de vitamine E et 5 à 50 mg de β-carotène par dose unitaire.

4. Composition pharmaceutique selon l'une des revendications 1 ou 3, caractérisée en ce qu'elle est sous forme de poudre, de comprimés, de gélules ou de capsules.

5. Composition pharmaceutique selon la revendication 4, caractérisée en ce qu'elle est sous forme de poudre pour solution buvable en sachet dose unitaire.

## Claims

1. Pharmaceutical composition comprising a mixture of D,L-lysine acetylsalicylate and racemic or dextrorotatory α-tocopherol, in base form or in the form of a salt, and β-carotene, in combination with suitable excipierts.

2. Pharmaceutical composition according to Claim 1, characterized in that the α-tocopherol is used in acetate, palmitate or succinate form.

3. Pharmaceutical composition according to either of Claims 1 and 2, characterized in that it contains 54 to 540 mg of DL-lysine acetylsalicylate, 10 to 10000 IU of vitamin E and 5 to 50 mg of β-carotene per single dose.

4. Pharmaceutical composition according to either of Claims 1 and 3, characterized in that it is in powder form or in the form of tablets, gelatin capsules or wafer capsules.

5. Pharmaceutical composition according to Claim 4, characterized in that it is in powder form for a drinkable solution as a single-dose sachet.

## Patentansprüche

1. Pharmazeutische Zubereitung, dadurch gekennzeichnet, daß sie aus einer Mischung aus D,L-Lysin-acetylsalicylat, racemischem oder rechtsdrehendem α-Tocopherol in Form der Base oder eines Salzes und β-Carotin in Kombination mit geeigneten Trägermaterialien gebildet ist.

2. Pharmazeutische Zubereitung nach Anspruch 1, dadurch gekennzeichnet, daß α-Tocopherol in Form des Acetats, Palmitats oder Succinats eingesetzt wird.

3. Pharmazeutische Zubereitung nach einem der Ansprüche 1 oder 2, dadurch gekennzeichnet, daß sie 54 bis 540 mg D,L-Lysin-acetylsalicylat, 10 bis 10000 IE Vitamin E und 5 bis 50 mg β-Carotin pro Einheitsdosis enthält.

4. Pharmazeutische Zubereitung nach einem der Ansprüche 1 oder 3, dadurch gekennzeichnet, daß sie in Form eines Pulvers, von Tabletten, Gelkapseln oder Kapseln vorliegt.

5. Pharmazeutische Zubereitung nach Anspruch 4, dadurch gekennzeichnet, daß sie in Form eines Pulvers für trinkbare Lösungen in einem Einheitsdosis-Sachet vorliegt.
